# EUROPEAN PATENT SPECIFICATION

(11) **EP 2 048 223 B1**
(45) Date of publication and mention of the grant of the patent: **25.03.2015**
(21) Application number: 07744392.7
(22) Date of filing: 30.05.2007
(51) Int. Cl.: C12M 3/00, C12M 3/04, C12Q 1/02, G01N 33/15, G01N 33/50

(54) **CELL CHIP**
ZELLCHIP
PUCES À CELLULES

(30) Priority: 30.05.2006 JP 2006149284
(43) Date of publication of application: 15.04.2009
(73) Proprietor: FUJIFILM Corporation, Minato-ku Tokyo 106-8620 (JP)
(72) Inventor: TANAKA, Masaru c/o Research Institute for Electric Science, Nishi 10-chome Kita-ku, Sapporo-shi, Hokkaido 001-0021 (JP); TSUKIYAMA, Shusaku c/o Creative Research Initiative"Sousei" National, Kita 21-jyo Nishi 10-chome Kita-ku sapporo-shi, Hokkaido 001-0021 (JP); SHIMOMURA, Masatsugu c/o Research Institute for Electric Science National, Kita 21-jyo Nishi 10-chome Kita-ku, Sapporo-shi, Hokkaido 001-0021 (JP); YAMAZAKI, Hidekazu, Minamiashigara-shi, Kanagawa 2500193 (JP); NARUSE, Hideaki, Minamiashigara-shi, Kanagawa 2500193 (JP); YAMAMOTO, Sadaaki c/o Creative Research Initiative"Sousei" National, Kita 21-jyo Nishi 10-chome Kita-ku sapporo-shi, Hokkaido 001-0021 (JP)
(74) Representative: Hoffmann Eitle
(86) International application number: PCT/JP2007/060979
(87) International publication number: WO 2007/139144

(56) References cited:
- JP-A- 2002 335 949
- TANAKA M ET AL: "Control of hepatocyte adhesion and function on self-organized honeycomb-patterned polymer film", COLLOIDS AND SURFACES. A, PHYSICACHEMICAL AND ENGINEERING ASPECTS, ELSEVIER, AMSTERDAM, NL, vol. 284-285, 9 February 2006 (2006-02-09) , pages 464-469, XP025136630, ISSN: 0927-7757, DOI: DOI:10.1016/J.COLSURFA.2005.11.098 [retrieved on 2006-08-15]
- NISHIKAWA Y. ET AL.: 'Honeycomb-jo Takosei Film ni yoru Kansoshiki Keisei no Yudo' POLYMER PREPRINTS, JAPAN vol. 49, 2000, pages 3967 - 3968, XP003019616
- SUNAMI H. ET AL.: 'II. Saisei Igaku no Nanotechnology Honeycomb Film o Mochiita Tanpakushitsu Kyuchaku Oyobi Saibo Setchaku no Seigyo' REGENERATIVE MEDICINE vol. 5, 2006, pages 96 - 101, XP003019617
- GUILLOUZO A. ET AL.: 'Use of human hepatocyte cultures for drug metabolism studies' TOXICOLOGY vol. 82, 1993, pages 209 - 219, XP003019618
- FUKUHIRA Y. ET AL.: 'Biodegradable honeycomb-patterned film composed of poly(lactic acid) and dioleoylphosphatidylethanolamine' BIOMATERIALS vol. 27, March 2006, pages 1797 - 1802, XP005204033
- YAMAMOTO S. ET AL.: 'Tokushu Mirai e Tsunagu Daigakuhatsu Venture Jiko Soshikika Micro Pattern Kobunshi Usumaku no Kaihatsu to Kodo Saisei Iryo eno Oyo' KAGAKU KOGYO vol. 57, 2006, pages 27 - 35, XP003019619
- SALONEN J.S. ET AL.: 'Comparative studies on the cytochrome p450-associated metabolism and interaction potential of selegiline between human liver-derived in vitro systems' DRUG METAB. DISPOS. vol. 31, 2003, pages 1093 - 1102, XP003019620
- TANAKA M. ET AL.: 'Effect of pore size of self-organized honeycomb-patterned polymer films on spreading, focal adhesion, proliferation, and function of endothelial cells' J. NANOSCI. NANOTECHNOL. vol. 7, March 2007, pages 763 - 772, XP003019621

## Description

### Technical Field

The present invention relates to a biomaterial utilizing a honeycomb-like porous material, and more particularly, to a cell chip formed from a honeycomb-like porous material and hepatocytes.

### Background Art

As one of the means or technologies effective for searching and identifying useful genes, there may be mentioned a means called a high density cDNA array, or a DNA chip. The DNA chip is a beneficial technology which makes it possible to analyze several thousand to several ten thousand genes simultaneously, and is also capable of providing highly reliable information in the clinical applications such as clinical diagnosis and prognosis.

Previous research including this DNA chip have mainly employed techniques of disrupting cells to separate DNAs or proteins from each other and detect them, or tracing the reactions. However, since these biopolymers intracellularly form well-ordered interactions with a wide variety of biological molecules that are present in the vicinity, and also, since the biopolymers are disposed at certain locations within the cells, it cannot be ascertained that the results of observation made on the behavior of the biomolecules obtainable by disrupting cells necessarily reflect the responses directly at the cellular level.

It is fundamentally desirable to observe the behavior of biomolecules using the cells themselves, and bio-cell chips in which cells are aligned and fixed on a chip substrate have also been developed (Patent Document 1). However, it is not easy in general to culture cells having specific functions, such as hepatocytes or neurons, for a long time period, and to retain the functions of the cells for that period. For example, cultured hepatocytes are being widely used to perform the screening of pharmaceutical products. However, conventional primary culture systems of hepatocytes have a time period for retaining the hepatocyte functions of as short as 1 to 2 days, and are low in function as compared with the hepatic tissue in a living body, and not were practical (Non-Patent Document 1).

It is expected that development of a technology for detecting various changes or interactions of biomolecules in a cell, without destroying the cell under observation, while retaining the interactions of the biopolymers in the cell, would lead to a high possibility of obtaining new findings that are previously unheard of.

As to such expectations, a technology of retaining living cells on a substrate while maintaining the cellular functions has been reported (for example, Non-Patent Documents 2 to 7). However, basically, retaining cells alive on a substrate while maintaining the cellular functions is generally never easy, as is the case with the previously mentioned retainment of the functions of cultured cells, and it is still difficult to obtain human hepatocytes having the cellular functions retained over a long time, even with the technologies described in the Non-Patent Documents 2 to 7.

Furthermore, as a material which has a potential to serve as a good scaffold for cell proliferation, there is known a honeycomb-like porous material formed from a water-insoluble polymer (Patent Document 2). However, it is not reported as to whether the proliferated cells would retain the functions over a long time, and it is not reported that this honeycomb-like porous material could be used as a substrate for a cell chip which is capable of retaining the cellular functions over a long time.

Tanaka, M., et al. (2006) describes the control of hepatocyte adhesion and function on self-organized honeycomb-patterned polymer film.

Nishikawa, Y., et al. (2000) describes culturing hepatocytes on a honeycomb-patterned polymer film.

Sunami, H., et al. (2006) describes the control of protein absorption and cell adhesion by using honeycomb film.

Guillouzo, A., et al. (1993) describes the investigation of drug metabolism using human hepatocyte cultures.

JP 2002-335949 A describes the cell three-dimensional tissue culture method using honeycomb structure film.
Non-Patent Document 1: Landry, J., et al., J. Cell Biol., Vol. 101, No. 3, pp. 914-923 (1985)
Non-Patent Document 2: Koide, N., et al., BBRC, Vol. 161, No. 1, pp. 385-391 (1982)
Non-Patent Document 3: Koide, N., et al., Exp. Cell Res., Vol. 186, No. 2, pp. 227-235 (1990)
Non-Patent Document 4: Tong, J.Z., et al., Exp. Cell Res., Vol. 200, No. 2, pp. 326-332 (1992)
Non-Patent Document 5: Matsushita, T., et al., Appl. Microbiol. Biotechnol., Vol. 36, No. 3, pp. 324-326 (1991)
Non-Patent Document 6: Park I.K., et al., Biomaterial, Vol. 24, No. 13, pp. 2331-2337 (2003)
Non-Patent Document 7: Fukuda, J. et al., Cell Transplant, Vol. 12, No. 1, pp. 51-58 (2003)
Patent Document 1: JP-W No. 2005-517411
Patent Document 2: JP-A No. 2001-157574

### Disclosure of the Invention

### Problems to be Solved by the Invention

The inventors of the present invention found that a honeycomb-like porous material formed from a water-insoluble polymer can, when used as a substrate for cell chips, retain human hepatocytes for a short time, while maintaining the drug metabolizing enzyme activities over a long time, and thus completed the following inventions as defined in the claims.

### Means for Solving the Problems

The description discloses
(1) A cell chip comprising a honeycomb-like porous thin film consisting of a water-insoluble polymer having a film thickness of 0.01 µm to 100 µm and through-pores with a pore diameter of 0.01 µm to 100 µm, wherein hepatocytes are retained on one surface or both surfaces of the thin film.
(2) The cell chip according to (1), wherein the hepatocytes are human hepatocytes.
(3) The cell chip according to (1) or (2), wherein the honeycomb-like porous thin film is a honeycomb-like porous material having a structure in which the through-pores are in communication with the through-pores in the vicinity occurring in the planar direction of the thin film.
(4) A method for evaluating the inhibitory capacity, suppressive capacity or activating capacity of a compound against the physiological activity of a target substance contained in hepatocytes by using the cell chip according to any one of (1) to (3), wherein the method comprises a process of incubating the cell chip and the compound together, and a process of measuring the physiological activity of the target substance contained in the hepatocytes which have been retained in the cell chip after the incubation.
(5) The method according to (4), wherein the target substance is P450.
The invention is described by the claims.

### Effects of the Invention

The cell chip retaining hepatocytes of the present invention, particularly the cell chip retaining human hepatocytes on both surfaces of a honeycomb-like porous material, has advantages such as that the cell chip can retain drug metabolizing enzyme activities, particularly P450 activity, over one week or longer, and stably maintains the amount of DNA, that is, the number of cells, for that period of time. In the case of using conventionally cultured cells, such maintaining of functions was achieved only for 1 to 2 days. Therefore, the ability to maintain cellular functions of the present invention is outstandingly excellent, and the cell chip of the present invention is effective for the screening of drugs using hepatocytes, and for the observation of the physiological influence by a compound on hepatocytes, or the like.

### Best Mode for Carrying Out the Invention

The cell chip according to the present invention means a biochip in which intact living cells are aligned and retained on a substrate, preferably a thin film. The term "retain" as used herein refers not to a state in which the cells and the substrate or the like are simply in contact, but to a state in which the cells are firmly fixed to the extent that even if the cells on the substrate are washed with water, buffer solution or the like, the cells are not easily detached from the substrate or the like.

The cell chip of the present invention can be produced by retaining hepatocytes, particularly human hepatocytes, to one surface or both surfaces of a honeycomb-like porous material (also referred to as honeycomb-structured material or honeycomb sheet) consisting of a water-insoluble polymer.

Here, the honeycomb-like porous material consisting of a water-insoluble polymer means a porous thin film made of a polymer, in which minute pores directed in the perpendicular direction of the membrane are provided in a honeycomb shape in the direction of the planar direction of the membrane. The honeycomb-like porous material used in the present invention has pores which penetrate through the film in the perpendicular direction (through-pores), and it is particularly preferable to use a honeycomb-like porous material in which the through-pores are in mutual communication with through-pores in the vicinity which occur in the planar direction.

Such porous thin film in which pores are provided in a regular disposition called a honeycomb pattern, is to be understood as a structure which is completely different from conventional porous materials having irregular pores with varying pore diameter, shape, depth or the like. It may be conceived by inference that the structure in which the through-pores occurring in the planar direction of the honeycomb-like porous material are in communication with each other, bring about advantages to the cell chip of the present invention, such as that an interaction between human hepatocytes retained in the honeycomb-like porous material, or supply of medium or dissolved oxygen and discharge of waste products are achieved smoothly.

The honeycomb-like porous material that can be used in the present invention has a shape in which the film thickness is 0.01 µm to 100 µm, preferably 0.1 µm to 50 µm, and more preferably 1 µm to 20 µm, and the pore diameter is 0.01 µm to 100 µm, preferably 0.1 µm to 50 µm, more preferably 1 µm to 20 µm, and particularly preferably 5 µm to 10 µm.

The honeycomb-like porous material having such structural features can be produced according to various known methods. For example, there may be mentioned photolithography or soft lithography (Whitesides, et al., Angew. Chem. Int. Ed., Vol. 37, pp. 550-575 (1998)), phase separation of a block copolymer (Albrecht, et al., Macromolecules, Vol. 35, pp. 8106-8110 (2002)), a method of producing a two-dimensional or three-dimensional periodic structure by agglomerating colloidal microparticles of submicron size (Gu, et al., Langmuir, Vol. 17), a method of producing an inversed opal structure using the periodic structure as a template (Kalso, et al., Langmuir, Vol. 15, pp. 8276-8281 (1999)), and the like.

The methods described JP-A No. 8-311231, JP-A No. 2001-157475, JP-A No. 2002-347107 or JP-A No. 2002-335949, which methods are largely different from the aforementioned methods in the principle of production, can also be used. These methods involve condensing water droplets on the surface of a solution of a polymer in a water-insoluble organic solvent, and preparing a honeycomb-like porous material by using the water droplets as a template. These methods are advantageous compared with other production methods in terms of the production costs or efficiency. Hereinafter, the methods will be described in more detail.

In this method, it is preferable that a solution of a water-insoluble polymer in a water-insoluble organic solvent in which a water-insoluble polymer is dissolved in a water-insoluble organic solvent, particularly a water-insoluble organic solvent having a surface tension, γL, of 50 dyn/cm or less, be applied on the surface of a substrate which satisfies the relationship of γS - ySL > γL, wherein γS represents the surface tension of the surface, and γL represents the surface tension of the applied water-insoluble organic solvent, and γLS represents the surface tension between the substrate and the solvent, and further, the solution of a water-insoluble polymer in a water-insoluble organic solvent applied on the substrate be evaporated in the presence of air at a relative humidity of 30% or higher.

The water-insoluble organic solvent as used herein refers to an organic solvent having a surface tension of 50 dyn/cm or less, water-insolubility to the extent that water droplets condensed on the solution surface may be retained, and a boiling point of 0 to 150°C, preferably 10 to 90°C, under the atmospheric pressure. For example, halogenated hydrocarbons such as carbon tetrachloride, dichloromethane and chloroform; aromatic hydrocarbons such as benzene, toluene and xylene; esters such as ethyl acetate and butyl acetate; water-insoluble ketones such as methyl isobutyl ketone; carbon disulfide; and the like may be mentioned.

Furthermore, the water-insoluble polymer is not particularly limited as long as it is a polymer which is insoluble in water and soluble in the above-mentioned water-insoluble organic solvents, or which can be dissolved in a water-insoluble organic solvent in the presence of an appropriate surfactant, and the polymer can be appropriately selected and used.

For example, a biodegradable polymer such as polylactic acid or polyhydroxybutyric acid, an aliphatic polycarbonate, an amphiphilic polymer, a photofunctional polymer, an electrofunctional polymer, and the like can be mentioned.

As an example of specific combination of the water-insoluble organic solvent and a water-insoluble polymer, for a polymer selected from the group consisting of, for example, polystyrene, polycarbonate, polysulfone, polyethersulfone, polyalkylsiloxane, polyalkyl methacrylate such as polymethyl methacrylate, or polyalkyl acrylate, polybutadiene, polyisoprene,
and copolymers thereof, an organic solvent such as carbon tetrachloride, dichloromethane, chloroform, benzene, toluene, xylene or carbon disulfide can be used in combination. Furthermore, for a polymer selected from the group consisting of acrylates, methacrylates and copolymers thereof, which respectively have fluorinated alkyl in the side chain, use of a carbon fluoride solvent such as AK-225 (manufactured by Asahi Glass Co., Ltd.), trifluorobenzene, fluoroethers or the like also yield good results. The solvent can be appropriately selected and used from among them, by specifically taking into consideration the solubility for a water-insoluble polymer to be used.

Furthermore, in the case of producing a honeycomb-like porous material using a fluorine-based polymer which is obtained by substituting the hydrogen in the side chain of a polyacrylate or methacrylate having fluorinated alkyl in the side chain, with fluorine, use of a fluorine-based organic solvent (AK-225 or the like) also gives good results.

The concentration of the water-insoluble polymer in the solution of a water-insoluble organic solvent can be appropriately determined depending on the characteristics and properties required for the honeycomb-like porous material to be produced, and the water-insoluble organic solvent. In the case of producing the honeycomb-like porous material having through-pores to be used in the present invention, it is preferable to set the concentration of the water-insoluble polymer in the solution of a water-insoluble organic solvent at 0.1 g/L to 10 g/L, and particularly at 0.5 g/L to 6.0 g/L.

Furthermore, as for the substrate which is coated with such solution of a water-insoluble polymer in a water-insoluble organic solvent, it is desirable to select and use a substrate which satisfies the relationship of γS - ySL > γL, wherein γS represents the surface tension of the substrate surface, γL represents the surface tension of the water-insoluble organic solvent to be applied, and γLS represents the surface tension between the substrate and the solvent. This is because the wettability of the substrate itself which is coated with the solution of a water-insoluble polymer in a water-insoluble organic solvent, may exert influence to the thickness of the liquid film formed on the substrate. It is preferable that the substrate is one having high affinity to the solution of a water-insoluble polymer in a water-insoluble organic solvent to be applied. Specifically, it is desirable to use a substrate having a surface exhibiting a surface tension which can be represented by the above-mentioned formula using the surface tension γL of the water-insoluble organic solvent as an index. As suitable examples of such substrate, there may be mentioned a glass plate, a plate made of silicone, a metal plate, or the like.

It is also possible to use a substrate, wherein the surface has been processed to enhance the affinity to the solution of a water-insoluble organic solvent. Such amelioration of the wettability of the substrate surface can be achieved by methods that are known per se, in accordance with the water-insoluble organic solvent used with the substrate, and for example, for a substrate made of glass or metal, by using a method of silane coupling treatment, a method of treatment by forming a monomolecular layer with a thiol compound, or the like.

For example, as for the substrate in the case of using a hydrophobic organic solvent such as chloroform as the water-insoluble organic solvent, a sufficiently washed Si substrate, or a glass substrate having the surface modified with an alkylsilane coupling agent or the like is preferably used. Also, in the case of using a fluorine-based solvent, a Teflon (registered trademark) substrate, or a glass substrate modified with a fluorinated alkylsilane coupling agent or the like is preferably used.

When the solution of a water-insoluble polymer in a water-insoluble organic solvent is applied on the substrate to form a coating film of the said solution, the liquid film thickness is preferably set to 1 µm to 1000 µm, and preferably 700 µm or less. Furthermore, as for the method of applying the solution of a water-insoluble polymer in a water-insoluble organic solvent on the substrate, there may be mentioned, in addition to a method of dropping the said solution on the substrate, a bar coating method, a dip coating method, a spin coating method and the like, and the methods can be used in either a batch mode or a continuous mode.

By evaporating the water-insoluble organic solvent from the thin film thus provided on the substrate, a honeycomb-like porous material formed from a water-insoluble polymer can be produced. In doing so, the pore diameter of the honeycomb-like porous material can be controlled by controlling the evaporation rate of the solvent.

The evaporation rate of the solvent can be controlled by placing the thin film on the substrate under an air stream having a relative humidity of 30% or higher at a wind velocity of 0.01 to 20 m/sec. In the production of the honeycomb-like porous material having through-pores that is used in the present invention, it is preferable to place the liquid film of the solution of a water-insoluble polymer in a water-insoluble organic solvent under an air stream adjusted to have a relative humidity in the range of 30 to 99% and a wind velocity in the range of 0.01 to 20 m/sec. In regard to the way of disposing the thin film against the direction of air stream, in a disposition established such that the thin film on the substrate is hit by the air stream from an obliquely upper direction or from a perpendicular direction, there may occur distortion or cracks in the thin film because of the wind pressure exerted by the air stream. In such cases, it is preferable to allow the thin film of the organic solvent solution on the substrate to be generated in parallel or in an upward direction with respect to the air stream. In this case, the air stream may be generated by any of a positive pressure from an upper stream or a negative pressure from a lower stream. For example, the process may be carried out in any manner, such that a predetermined amount of air may be jetted out from nozzles installed toward the substrate, or air in the upper part of the substrate may be aspirated from one direction.

In the present disclosure, the cell chip can be produced by disseminating and retaining hepatocytes on both surfaces of the honeycomb-like porous material prepared according to the above-described methods. Since conventional cell chips have a large number of cells aligned and fixed in a narrow space, special measures are required in order to prevent the cell suspension preserved in the fixative from spreading. However, the present disclosure is not subject to such limitation, and an operation of placing hepatocytes on the honeycomb-like porous material for an appropriate time period may be carried out for the both surfaces of the honeycomb-like porous material.

Specifically, a honeycomb-like porous material which has been disinfected by heat sterilization or ultraviolet irradiation is immersed in an appropriate medium selected from, for example, DMEM, F-12, William's Medium E and the like, and 0.5 × 10⁵ to 1.0 × 10⁵ hepatocytes suspended in the said medium are disseminated on the immersed honeycomb-like porous material. Then, the system is left to stand under usual conditions for cell culture for about 3 to 5 hours. Thereafter, the front and back sides of the honeycomb-like porous material, may be turned over to repeat the same operation.

As for the hepatocyte, hepatocytes derived from mammals, for example, various animals represented by rat, mouse, rabbit and the like, can all be used, but it is particularly preferable to use human hepatocyte. In general, it is understood that even if hepatocytes collected from a living body are preserved in an appropriate medium, or the like, it is difficult to retain the cellular functions. For example, if collected human hepatocytes are placed in an appropriate medium, the cellular functions are largely reduced in a period of about three days, and as the time of preservation further elapses, the cellular functions are continuously reduced. Particularly, activity reduction in the group of proteins involved in the drug metabolizing activity, inter alia, P450, makes it difficult to perform the experiment for evaluation of drug candidate compounds using human hepatocytes. It is because reduction of the drug metabolizing activity of human hepatocytes means that the operation of the experiment for evaluation using human hepatocytes should be completed within one day to two days from the preparation of human hepatocytes.

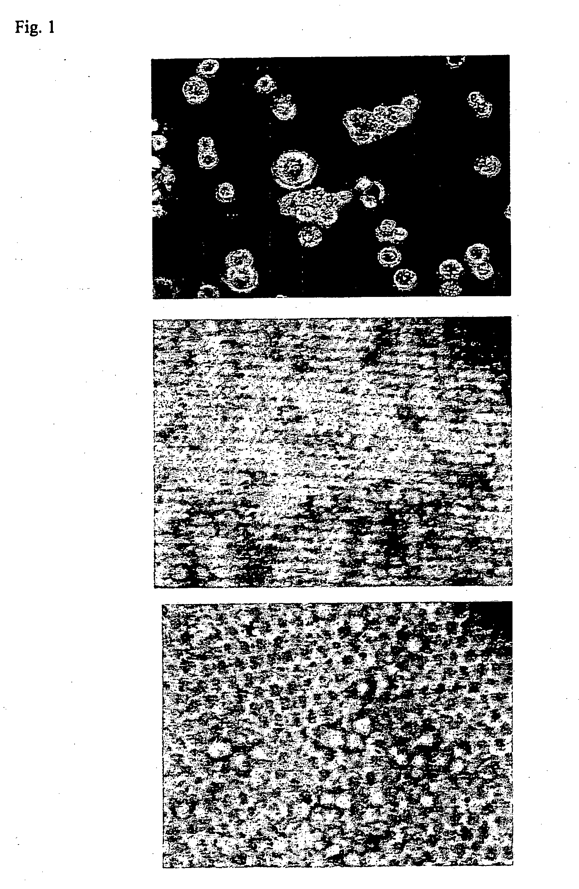

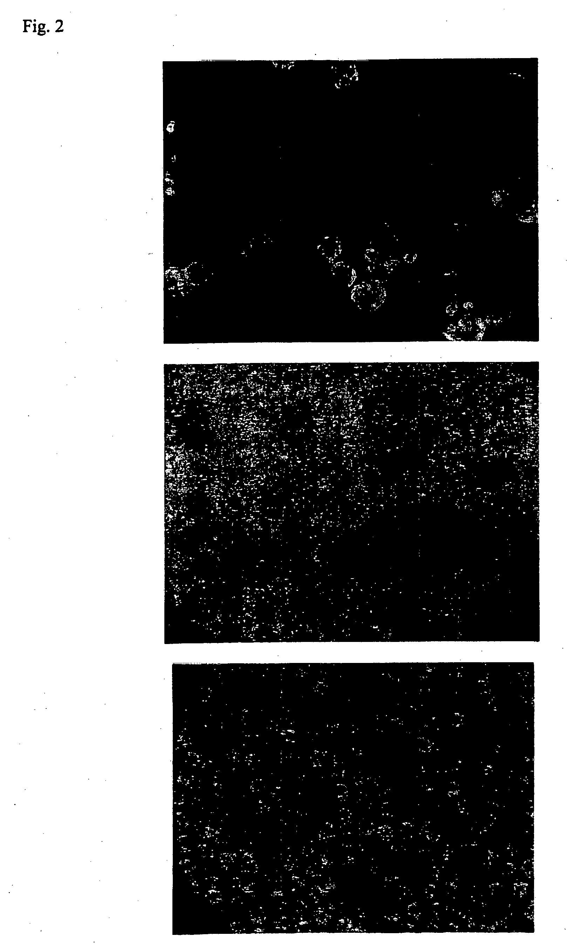

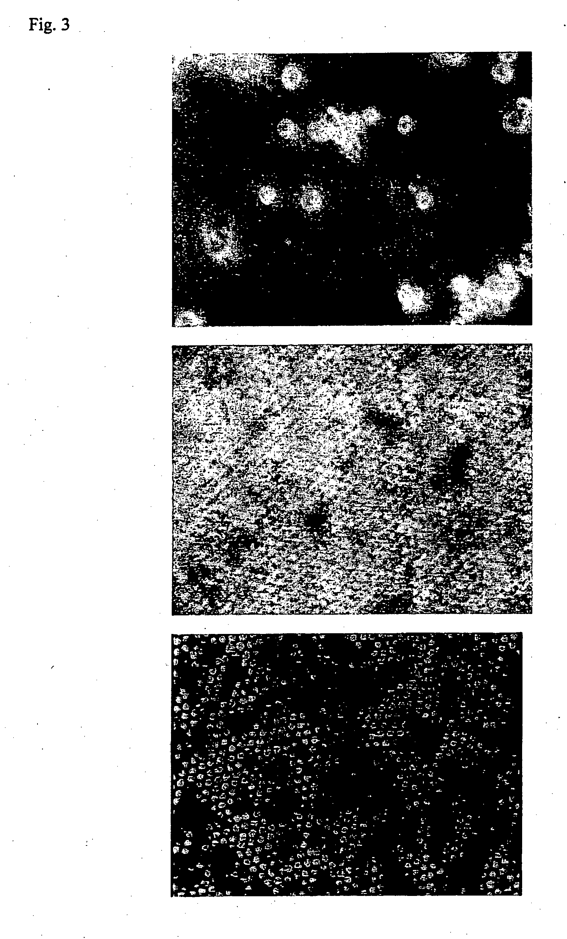

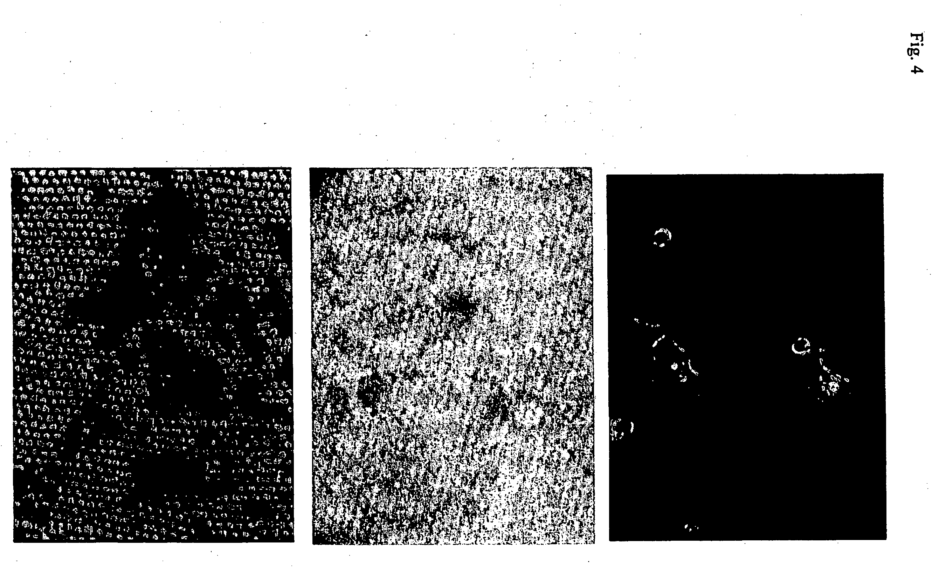

Meanwhile, in regard to the cell chip of the present invention, as will be shown in the following Examples, after disseminating human hepatocytes on the honeycomb-like porous material, reduction of cellular functions is observed up to 3 days; however, the reduction of cellular functions due to the lapse of preservation time thereafter is not observed over 7 days in most of the cases (Figs. 1 to 4). The cell chip of the present invention therefore solves the restriction that the operation of the experiment for evaluation using human hepatocytes as described above should be completed within 1 day to 2 days from the preparation of the human hepatocytes. Furthermore, since the human hepatocytes retained on the cell chips of the present invention are hardly lost by suspending, the cell chip of the present invention can supply a stable number of human hepatocytes.

Only by presumption, it is conceived that the human hepatocytes attached onto both surfaces of the honeycomb-like porous material having through-pores reproduce the peripheral environment (three-dimensional environment) of human hepatocytes in vivo in a quasi manner, by exerting certain interaction through the through-pores and/or through the parts communicating with through-pores in the vicinity occurring in the planar direction, and as a result, effects are manifested such as that the adhesiveness of the human hepatocytes to the honeycomb-like porous material is enhanced, and the deterioration of cellular functions is suppressed.

The cell chip of the present invention having the above-described advantageous features also provides a method of evaluating the inhibitory capacity, suppressive capacity or activating capacity of a compound against the physiological activity of a target substance contained in hepatocytes by using the subject cell chip, wherein the method comprises a process of incubating the cell chip and the compound together, and a process of measuring the physiological activity of the target substance contained in the hepatocytes which have been retained in the cell chip after the incubation. It is particularly preferable for the method that the target substance is P450.

Specifically, the cell chip of the present invention is placed in an appropriate liquid medium or buffer solution, wherein the compound is added thereto in an appropriate amount, and the system is incubated. Then, the amount or physiological activity of the target substance contained in the hepatocytes, typically of a protein associated with metabolism and other cellular functions, is measured to determine whether the added compound would inhibit, suppress or activate the expression or activity of the protein provided as the target substance, by comparing with the results obtained by using an appropriate control substance.

Selection of the target substance is completely at discretion, and the evaluation of the amount or physiological activity of the target molecule may be performed by employing the respective methods defined for various target substance. In regard to the cell chip retaining human hepatocytes of the present invention, preferred target molecules are a group of proteins involved in drug metabolism, and a particularly preferred protein is P450.

### Brief Description of the Drawings

Fig. 1 shows photographs obtained by disseminating human hepatocytes, and then observing the cells on various films on the first day by phase contrast inverted microscopic photography. The photographs represent, from the top, a smooth film, a non-penetrating film, and a honeycomb film.
Fig. 2 shows photographs obtained by disseminating human hepatocytes, and then observing the cells on various films on the third day by phase contrast inverted microscopic photography. The photographs represent, from the top, the smooth film, the non-penetrating film, and the honeycomb film.
Fig. 3 shows photographs obtained by disseminating human hepatocytes, and then observing the cells on various films on the fifth day by phase contrast inverted microscopic photography. The photographs represent, from the top, the smooth film, the non-penetrating film, and the honeycomb film.
Fig. 4 shows photographs obtained by disseminating human hepatocytes, and then observing the cells on various films on the seventh day by phase contrast inverted microscopic photography. The photographs represent, from the top, the smooth film, the non-penetrating film, and the honeycomb film.
Fig. 5 is a graph showing the results of measuring the activity of a drug metabolizing enzyme, P450, in (1) of Test Example. The bars in the graph indicate, from the left-hand side for each of the films, the first day, third day, fifth day and seventh day from dissemination.
Fig. 6 is a graph showing the results of measuring the amount of DNA in (2) of Test Example. The bars in the graph indicate, from the left-hand side for each of the films, the first day, third day, fifth day and seventh day from dissemination.

Hereinafter, the present invention will be described in more detail by referring to Examples, but the present invention is not intended to be limited to these Examples.

### EXAMPLE 1

### (1) Production of honeycomb-like porous material

Poly(ε-caprolactone) (PCL, manufactured by Wako, molecular weight 70,000 to 100,000), which is a biodegradable polymer, and an amphiphilic polyacrylamide polymer (Cap) were dissolved in chloroform at a weight ratio of 10 : 1 to a concentration of 0.5 mg/mL. This solution was cast on a glass petri dish (φ 90 mm), and chloroform was evaporated in an atmosphere at a relative humidity of 80%. Thereby, a honeycomb-like porous material (honeycomb film) (φ 90 mm) having pores with a pore diameter of 7 to 9 µm penetrating through the membrane was produced.

Furthermore, as a comparative object, a honeycomb-like porous material (non-penetrating film) (φ 90 mm) having, on one side thereof, dents with a pore diameter of 6 to 8 µm, which did not penetrate through the membrane, was produced as in the above-described method, by casting a chloroform solution of PCL at a concentration of 5 mg/mL on a glass petri dish, and evaporating chloroform in an atmosphere at a relative humidity of 80%. In addition, 20 µL of this chloroform solution was cast on a cover glass, and then the cover glass was rotated in a spin coater (MIKASA 1H-D7) at 1000 rpm for 30 seconds to cover the entire surface with the polymer, to thereby produce a poreless flat form (smooth film) of 22 mm in size.

### (2) Culture of human hepatocytes

A vial of frozen human hepatocytes (BD Biosciences, USA) (number of cells 5.8 × 10⁶ cells/vial) was lysed at 37°C, and the cells were recovered by a density gradient method using a cryopreserved hepatocyte purification kit (BD Biosciences, USA). The cells were suspended in a DMEM medium (Invitrogen, USA) containing 10% FBS, dexamethasone, insulin, nicotinamide and ascorbic acid, to prepare a cell suspension.

### (3) Pretreatment of films

The smooth film, the non-penetrating film and the honeycomb film were sterilized by irradiating the films with an ultraviolet ray of 253.7 nm for 2 hours or longer. After the sterilization, the respective films were placed one sheet each in the wells of a 12-well plate, and for the smooth film and the non-penetrating film, presser glass rings were mounted on the films. Subsequently, ethanol was added to degas, the medium was replaced with sterilized Milli Q water, and then replaced with DMEM medium. All of the films were left to stand for 2 hours or longer with the medium introduced therein, and then were used in the subsequent operation.

### (4) Dissemination of hepatocytes

The cell suspension prepared in (2), having the number of cells shown in the table below, was disseminated on the respective films prepared in (3), and the cells were cultured in an incubator at 37°C and 5% CO₂. For the honeycomb film, a half the number of cells shown in the table below were disseminated on one side, and after 5 hours, the film was reversed to disseminate the remaining cells on the other side.

**[Table 1]**

| Type of film | Number of cells per sheet (cells) | Area of film (cm²) | Cell density (/cm²) | Remarks |
|---|---|---|---|---|
| Smooth film (PCL/PS) | 1.778×10⁵ | 2.54 | 0.7×10⁵ | |
| Non-penetrating film | 1.778×10⁵ | 2.54 | 0.7×10⁵ | |
| Honeycomb film | 0.56×10⁵ | 0.56 | 1.0×10⁵ | Sum of both sides |

On the first, third, fifth and seventh days from the dissemination, the culture vessels were gently shaken, and non-adhered cells were removed together with the medium. Thereby, cell chips having human hepatocytes retained on honeycomb films were obtained. The cells chips were preserved by adding DMEM medium thereto.

In the cell chip obtainable from the honeycomb film, more adhered cells were observed in comparison to the other two types (Fig. 1 to Fig. 4). In this cell chip, even with the elapse of the days, the appearance of floating cells was not recognized. In the cell chip obtainable from the non-penetrating film, there was a tendency that the number of adhered cells decreases along with the elapse of the days, as compared to the cell chip obtainable from the honeycomb film (Fig. 1 to Fig. 4). Furthermore, in the dissemination and culture of human hepatocytes on the smooth film, a sufficient number of adhered cells were not obtained (Fig. 1 to Fig. 4).

### <TEST EXAMPLE>

### (1) Measurement of P450 activity

Measurement was made of the activity of P450, a drug metabolizing enzyme, in the human hepatocytes present on the cell chips obtained in Example 1. The medium was removed from the cell chips, and the cell chips were washed two times with a KHB solution (Krebs-Henseleit Buffer, Sigma-Aldrich, USA). Subsequently, a 100 mM testosterone solution was added as the reaction substrate, in an amount of 0.5 ml per sheet of the films, and the cell chips were left to stand still in an incubator at 37°C and 5% CO₂. After the standing, the solution was recovered, and the content of 6β-hydroxytestosterone, which is a product of metabolism of testosterone by P450, in the solution was measured.

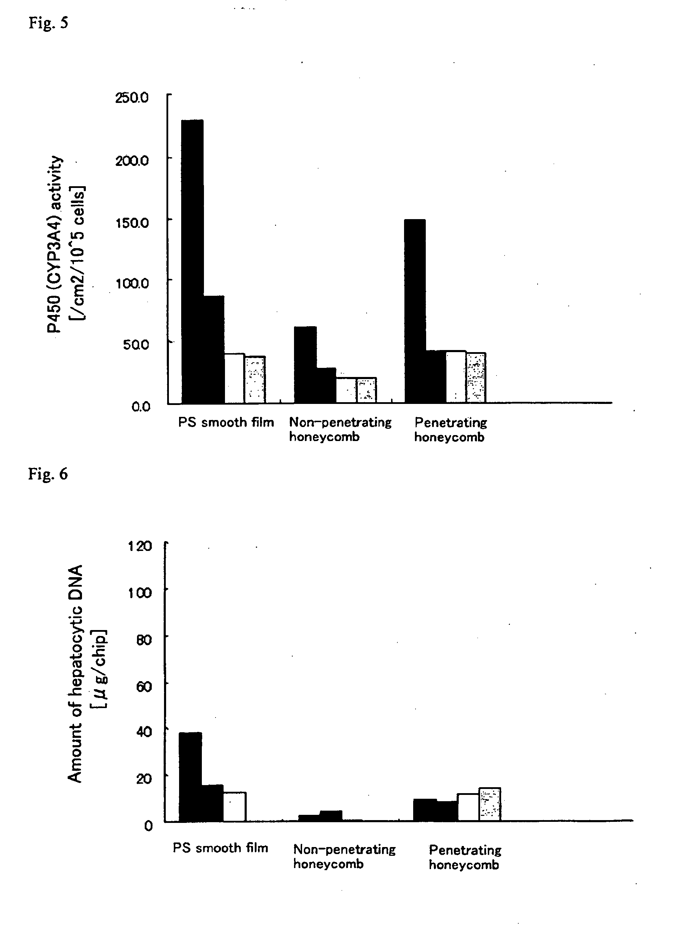

As a result, in the smooth film and the non-penetrating film, high P450 activity values were shown on the first day of culture, and then the activity was reduced along with the elapse of the days of culture, whereas in the cell chip obtainable from the honeycomb film, even though culturing was continued, nearly constant P450 activity was retained up to the seventh day (Fig. 5).

### (2) Measurement of amount of DNA

After recovering samples for the measurement of P450 activity, a Tris-EDTA-NaCl buffer solution was added to the culture vessels, and the cells were cryopreserved. After thawing, Proteinase K was added thereto so that a final concentration be 100 µM, and the culture vessels were kept warm at 37°C for 1 hour. Subsequently, the cells and the films were disrupted with an ultrasonic homogenizer, and centrifuged at 12,000 rpm at 4°C for 20 minutes to recover the supernatant. Hoechst 33258 was added as a color developing agent, and fluorescence was measured at 465 nm, with an excitation light of 340 nm, to thus calculate the amount of DNA.

As a result of this calculation, it was confirmed that in the cell chip obtainable from the honeycomb film, the amount of DNA was nearly constant over the third, fifth and seventh day of culture. On the other hand, in the smooth film and the non-penetrating film, there was recognized a tendency in which the amount decreased along with the elapse of the days of culture, and on the seventh day, the amount was less than the limit of detection.

## Claims

1. A cell chip for evaluating the inhibitory capacity, suppressive capacity or activating capacity of a compound against the physiological activity of a target substance contained in hepatocytes comprising a honeycomb-like porous thin film consisting of a water-insoluble polymer selected from the group consisting of polystyrene, polycarbonate, polysulfone, polyethersulfone, polyalkylsiloxane, polyalkyl methacrylate, polyalkyl acrylate, polybutadiene, polyisoprene, and copolymers thereof, the honeycomb-like porous thin film having a film thickness of 0.01 µm to 100 µm and through-pores with a pore diameter of 0.01 µm to 100 µm, wherein hepatocytes are retained on one surface or both surfaces of the thin film.

2. The cell chip according to claim 1, wherein the hepatocytes are human hepatocytes.

3. The cell chip according to claim 1 or 2, wherein the honeycomb-like porous thin film is a honeycomb-like porous material having a structure in which the through-pores are in communication with the through-pores in the vicinity occurring in the planar direction of the thin film.

4. The cell chip according to any one of claims 1 to 3, wherein the hepatocytes are retained on both surfaces of the thin film.

5. A method for evaluating the inhibitory capacity, suppressive capacity or activating capacity of a compound against the physiological activity of a target substance contained in hepatocytes by using the cell chip according to any one of claims 1 to 4, wherein the method comprises a process of incubating the cell chip and the compound together, and a process of measuring the physiological activity of the target substance contained in the hepatocytes which have been retained in the cell chip after the incubation.

6. The method according to claim 5, wherein the target substance is P450.

## Patentansprüche

1. Zell-Chip zur Beurteilung der inhibierenden Kapazität, unterdrückenden Kapazität oder aktivierenden Kapazität eines Stoffes gegen die physiologische Aktivität eines in Hepatozyten enthaltenen Zielstoffes, umfassend einen Honigwaben-artigen porösen dünnen Film, bestehend aus einem Wasser-unlöslichen Polymer ausgewählt aus der Gruppe bestehend aus Polystyrol, Polykarbonat, Polysulfon, Polyethersulfon, Polyalkylsiloxan, Polyalkylmethacrylat, Polyalkylacrylat, Polybutadien, Polyisopren und Kopolymeren davon, wobei der Honigwaben-artige poröse dünne Film eine Filmdicke von 0,01 µm bis 100 µm und durchgehende Poren (through-pores) mit einem Porendurchmesser von 0,01 µm bis 100 µm aufweist und wobei Hepatozyten auf einer Oberfläche oder beiden Oberflächen des dünnen Films festgehalten werden.

2. Zell-Chip gemäß Anspruch 1, wobei die Hepatozyten menschliche Hepatozyten sind.

3. Zell-Chip gemäß Anspruch 1 oder 2, wobei der Honigwaben-artige poröse dünne Film ein Honigwaben-artiges poröses Material ist, das eine Struktur aufweist, in der die durchgehenden Poren mit den durchgehenden Poren, die in der planaren Richtung des dünnen Films in der Nähe vorkommen, in Verbindung (in communication) stehen.

4. Zell-Chip gemäß einem der Ansprüche 1 bis 3, wobei die Hepatozyten auf beiden Oberflächen des dünnen Films festgehalten werden.

5. Verfahren zur Beurteilung der inhibierenden Kapazität, unterdrückenden Kapazität oder aktivierenden Kapazität eines Stoffes gegen die physiologische Aktivität eines in Hepatozyten enthaltenen Zielstoffes, durch Verwendung des Zell-Chips gemäß einem der Ansprüche 1 bis 4, wobei das Verfahren einen Prozess der Inkubation des Zell-Chips gemeinsam mit dem Stoff umfasst und einen Prozess des Messens der physiologischen Aktivität des in den Hepatozyten, die nach der Inkubation in dem Zell-Chip festgehalten wurden, enthaltenen Zielstoffes.

6. Verfahren gemäß Anspruch 5, wobei der Zielstoff P450 ist.

## Revendications

1. Puce à cellules permettant d'évaluer la capacité d'inhibition, la capacité suppressive ou la capacité d'activation d'un composé par rapport à l'activité physiologique d'une substance cible contenue dans des hépatocytes comprenant un film mince poreux en nid d'abeilles constitué d'un polymère insoluble dans l'eau choisi dans le groupe constitué de polystyrène, de polycarbonate, de polysulfone, de polyéthersulfone, de polyalkylsiloxane, de polyméthacrylate d'alkyle, de polyacrylate d'alkyle, de polybutadiène, de polyisoprène et de copolymères de ceux-ci, le film mince poreux en nid d'abeilles ayant une épaisseur de film allant de 0,01 µm à 100 µm et des pores traversants avec un diamètre de pore allant de 0,01 µm à 100 µm, où des hépatocytes sont retenus sur une surface ou sur les deux surfaces du film mince.

2. Puce à cellules selon la revendication 1, dans laquelle les hépatocytes sont des hépatocytes humains.

3. Puce à cellules selon la revendication 1 ou 2, dans laquelle le film mince poreux en nid d'abeilles est un matériau poreux en nid d'abeilles ayant une structure dans laquelle les pores traversants sont en communication avec les pores traversants à proximité qui apparaissent dans la direction plane du film mince.

4. Puce à cellules selon l'une quelconque des revendications 1 à 3, dans laquelle les hépatocytes sont retenus sur les deux surfaces du film mince.

5. Procédé permettant d'évaluer la capacité d'inhibition, la capacité suppressive ou la capacité d'activation d'un composé par rapport à l'activité physiologique d'une substance cible contenue dans des hépatocytes en utilisant la puce à cellules selon l'une quelconque des revendications 1 à 4, où le procédé comprend un processus d'incubation de la puce à cellules et du composé ensemble, et un processus de mesure de l'activité physiologique de la substance cible contenue dans les hépatocytes qui ont été retenus dans la puce à cellules après l'incubation.

6. Procédé selon la revendication 5, dans lequel la substance cible est P450.
